# EUROPÄISCHE PATENTSCHRIFT

(11) **EP 0 922 266 B1**
(45) Veröffentlichungstag und Bekanntmachung des Hinweises auf die Patenterteilung: **08.11.2000**
(21) Anmeldenummer: 97918910.7
(22) Anmeldetag: 27.08.1997
(51) Int. Cl.: G06F 19/00

(54) **VERFAHREN ZUR ERMITTLUNG SIGNIFIKANTER ABWEICHUNGEN DES ZELLENWACHSTUMS**
PROCESS FOR THE DETERMINATION OF SIGNIFICANT ANOMALIES IN CELL GROWTH
PROCEDE DE DETERMINATION D'ANOMALIES SIGNIFICATIVES DE LA CROISSANCE CELLULAIRE

(30) Priorität: 30.08.1996 DE 19635172; 26.08.1997 DE 19737109
(43) Veröffentlichungstag der Anmeldung: 16.06.1999
(73) Patentinhaber: PE Diagnostik GmbH, 09557 Flöha (DE)
(72) Erfinder: LEONHARDT, Peter, D-04177 Leipzig (DE); ROTZSCH, Wolfgang, D-04416 Markkleeberg (DE); LÖSER, Thomas, D-04105 Leipzig (DE); KELLER, Thomas, D-04275 Leipzig (DE); BITTERLICH, Norman, D-09113 Chemnitz (DE)
(74) Vertreter: Seerig, Dieter, Dipl.-Ing.
(86) Internationale Anmeldenummer: DE9701858
(87) Internationale Veröffentlichungsnummer: WO9809242

(56) Entgegenhaltungen:
- US-A- 5 499 319
- ARITA S ET AL: "Diagnostic system for diabetes mellitus based on the response of glucose tolerance test using a fuzzy inference" MEDINFO 92. PROCEEDINGS OF THE SEVENTH WORLD CONGRESS ON MEDICAL INFORMATICS, GENEVA, SWITZERLAND, 6-10 SEPT. 1992, ISBN 0-444-89668-6, 1992, AMSTERDAM, NETHERLANDS, NORTH-HOLLAND, NETHERLANDS, Seite 958 vol.2 XP002049352
- KUZNETSOV V A ET AL: "Syndrome approach for computer recognition of fuzzy systems and its application to immunological diagnostics and prognosis of human cancer" MATHEMATICAL AND COMPUTER MODELLING, MARCH 1996, ELSEVIER, UK, Bd. 23, Nr. 6, ISSN 0895-7177, Seiten 95-119, XP002049353
- SASAKI K ET AL: "A tuning treatment and application of fuzzy reasoning to freshness judgment of Hiroshima raw oysters" JAPANESE JOURNAL OF FUZZY THEORY AND SYSTEMS, 1993, USA, Bd. 5, Nr. 6, ISSN 1058-7349, Seiten 1009-1020, XP002049354

## Beschreibung

Die Erfindung betrifft ein Verfahren zur Ermittlung signifikanter Abweichungen des Zellenwachstums anhand gefundener Indikatorstoffe aus Körperflüssigkeiten .

Aus fachmännischen Kreisen ist bekannt, daß im Rahmen von Laboruntersuchungen von Körperflüssigkeiten Makromoleküle und Spurenelemente nachgewiesen werden, die aufgrund der veränderten Genexpression und anderer Faktoren entstehen, wobei sie von den Tumorzellen produziert werden, in anderen Körperzellen durch den Tumor induziert werden beziehungsweise als tumorunspezifische Stoffe in ihrer Konzentration durch den Tumor verändert werden. Der Nachweis solcher Stoffe in Körperflüssigkeiten gilt als Indikator für signifikante Abweichungen des Zellenwachstums. Die geringe Spezifität und Sensitivität genügt jedoch nicht den Anfordeungen für das Screening asymptomatischer Organismen. Deshalb beschränkt sich die Anwendung auf die Diagnostik von Patienten in Risikogruppen oder bei klinischer Symptomatik (vgl. Pathobiochemie und klinische Chemie, Verlag Wissenschaftliche Scripten 1955, Seite 93). Aus der DE 4110 217 C2 ist ein Verfahren zur Diagnose von Krankheitszuständen des menschlichen Organismus oder von Säugetieren unter Verwendung von Körperflüssigkeiten bekannt. Dieses Verfahren ist sehr aufwendig. Zuerst muß ein Kristallisat aus Venenblut oder Urin einer Person hergestellt werden. Um dieses Kristallcluster zu erzeugen, sind neben einer Vorrichtung zur Durchführung einer Wasserdampf-Mazeration noch weitere technische Einrichtungen, wie Tiefkühler, Erlenmeyerkolben, notwendig. Mit diesen technischen Mitteln verbinden sich eine Anzahl von Arbeitsschritten bei der Herstellung des Kristallclusters. Ist der Kristallcluster hergestellt, erfolgt die fotooptische Erfassung des als Kristallcluster ausgebildeten Kristallisates und Scannen des fotooptisch erfaßten Bildes. Das gescannte Bild wird nachfolgend digitalisiert, und die morphologische, colorimetrische und densiometrische Meßparameter werden unter Verwendung eines Bildanalysesystems ermittelt. Aus den einzelnen Meßparameter werden Datensätze gebildet, die zu einem Datenblock zusammengesetzt werden. Abschließend werden die Datensätze des Datenblockes mit in einer Datenbank erfaßten und aufbereiteten Datensätzen von Datenblöcken von Kristallclustern von Menschen oder Säugetieren, von denen diagnostisch gesicherte Befunde vorliegen, verglichen und der Kristallcluster aus den abgespeicherten Datenblöcken ermittelt, dessen Daten die höchste Übereinstimmung aufweisen.

Dokument ARITA S ET AL: "Diagnostic System for diabetes mellitus based on the response of glucose tolerance test using a fuzzy inference" MEDINFO 92. PROCEEDINGS OF THE SEVENTH WORLD CONGRESS ON MEDICAL INFORMATICS, GENEVA, SWITZERLAND, 6-10 SEPT. 1992, ISBN 0-444-89668-6, 1992, AMSTERDAM, NETHERLANDS, NORTH-HOLLAND, NETHERLANDS, Page 958 vol.2 bezieht sich auf eine Anwendung der Fuzzy Methodik zur Unterstützung der Diabetes-Diagnose. Dabei stützen sich die Autoren auf bekannte Meßverfahren und nutzen Entscheidungskriterien, wie sie international anerkannt sind (Vorschlag der WHO). Diese Kriterien finden im Bereich der festgelegten Schwellenwerte keine praktischen Akzeptanz, da geringe Änderungen des Meßwertes zu einer sprunghaften Entscheidungsänderung führen. Um diesem Effekt entgegenzuwirken, verwenden die Autoren die Theorie der Fuzzy Sets, die in sehr allgemeine Weise die Möglichkeit für eine mathematische Beschreibung von Übergangsverhalten bereithält. Gemäß des bislang üblichen Meßregimes werden die Meßwerte aufgenommen und für jeden Zeitpunkt gemäß der bekannten Entscheidungskriterien mit Hilfe von geeignet gewählten Zugehörigkeitsfunktionen die Aussage zur Diagnose gebildet. Zur zusammenfassenden Auswertung der Aussagen zu allen Zeitpunkten wird mit üblichen Methoden der Defuzzifizierung (hier Schwerpunktmethode) eine "mittlere" Diagnose ermittelt.

Dokument KUZNETSOV V A ET AL: "Syndrome approach for computer recognition of fuzzy systems and its application to immunological diagnostics and prognosis of human cancer" MATHEMATICAL AND COMPUTER MODELLING, MARCH 1996, ELSEVIER, UK, vol. 23, no. 6, ISSN 0895-7177, pages 95-119, XP002049353 bestätigt die Aktualität und die Notwendigkeit, die Entwicklung routinefähiger und praktikabler Verfahren zur Unterstützung der Tumordiagnostik und -Therapie zu forcieren. Die Autoren stellen ein Analyseverfahren vor, daß bei bekannter Diagnostik für eine ausgewählte Therapieform den Therapienerfolg bzw. die Metastasierung prognostizieren hilft. Mit der komplexen-Datenanalyse wird es den Autoren möglich keine Meßwerte aus der Fülle der Verfügbaren Daten zu erkennen, die eine Klassifikation in die Klassen "ungünstiger/günstiger Therapieverlauf" bzw. "mit/ohne Metastasierung" ermöglichen. Die Autoren verweisen jedoch darauf und die exemplarischen Daten in den veröffentlichten Tabellen-bestätigen auch, daß die statistische Analyse keine Unterschiede zu den "Normal-Gruppe nachzuweisen vermag.

Aufgabe der Erfindung ist es, ein Verfahren zur Ermittlung signifikanter Abweichungen des Zellenwachstums anhand gefundener Indikatorstoffe aus Körperflüssigkeiten zu entwickeln, das den Anforderungen an das Screening asymptomatischer Organismen entspricht und bei dem der technische Aufwand zur Ermittlung der Meßwerte bedeutend verringert ist.

Erfindungsgemäß wird die Aufgabe dadurch gelöst, daß für die nachfolgenden Verfahrensschritte Indikatorstoffe verwendet werden, die je nach ihrer Art von Tumorzellen produziert, in anderen Körperzellen durch den Tumor induziert und als tumorunspezifische Stoffe in ihrer Konzentration durch den Tumor verändert werden, sowie Meßwerte der Indikatorstoffe verwendet werden, die aus einer direkten Stoffanalyse ermittelt worden sind, und daß
(1) die Meßwerte der Indikatorstoffe in Datenblöcke zusammengefaßt werden,
(2) jeder Meßwert der Indikatorstoffe anhand gespeicherter und diagnostisch gesicherter Datenblöcke fuzzifiziert wird, um so eine Übereinstimmung in relativen Größen entsprechend der qualitativen Bewertung "normal", "unkritisch", "kritisch" ausdrücken zu können, und
(3) diese fuzzifizierten Datenblöcke mit den gespeicherten Datenblöcken verglichen, mittels Fuzzy-Entscheidungshilfesystem kombiniert und bezüglich der Charakteristika
   . Malignität
   . Histologie
   . Differentialhistologie
   einem Zahlenwert zwischen 0 und 1 zugeordnet werden.

Vorteilhaft ist es, daß die Indikatorstoffe Makromoleküle und Spurenelemente sind.
Weiterhin ist es zweckmäßig, daß die Indikatorstoffe Spurenelemente sind.
In einer weiteren Ausgestaltung der Erfindung sind die Indikatorstoffe M akromoleküle.

Die Erfindung wird anhand von vier Ausführungsbeispielen näher erläutert. Für jedes Beispiel werden aus Blut oder anderen Körperflüssigkeiten Makromoleküle und Spurenelemente als Indikatorstoffe verwendet, die je nach ihrer Art von Tumorzellen produziert werden, in anderen Körperzellen durch den Tumor induziert werden und als tumorunspezifische Stoffe in ihrer Konzentration durch den Tumor verändert werden. Die Meßwerte der Indikatorstoffe werden aus einer direkten Stoffanalyse ermittelt. In den Ausführungsbeispielen werden nachfolgend die weiteren Verfahrensschritte zur Ermittlung der signifikanten Abweichungen aufgezeigt.

### Ausführungsbeispiel 1:

(1) Die Meßwerte der Indikatorstoffe im Blutplasma einer Person werden gemäß erstem Verfahrensschritt in Datenblöcke zusammengefaßt. Im Ergebnis erhält man folgende Datenliste:

| Indikatorstoff | Meßwert |
|---|---|
| NSE (Neuronenspezifische Enolase) | 8.56 µg/l |
| CEA (Carzinoembryonales Antigen) | 2.07 µg/l |
| CYFRA21-1 (spezieller Tumormarker) | 1.48 µg/l |
| Kupfer-Konzentration (65_Cu) | 841 µg/l |
| Zink-Konzentration (66_Zn) | 785 µg/l |

(2) Im zweiten Verfahrensschritt wird jeder Meßwert der Indikatorstoffe anhand gespeicherter, diagostisch gesicherter Datenblöcke fuzzifiziert, um so die Übereinstimmung mit den Referenzbeispielen in relativen Größen entsprechend der qualitativen Bewertungen "normal", "unkritisch" bzw. "kritisch" ausdrücken zu können: 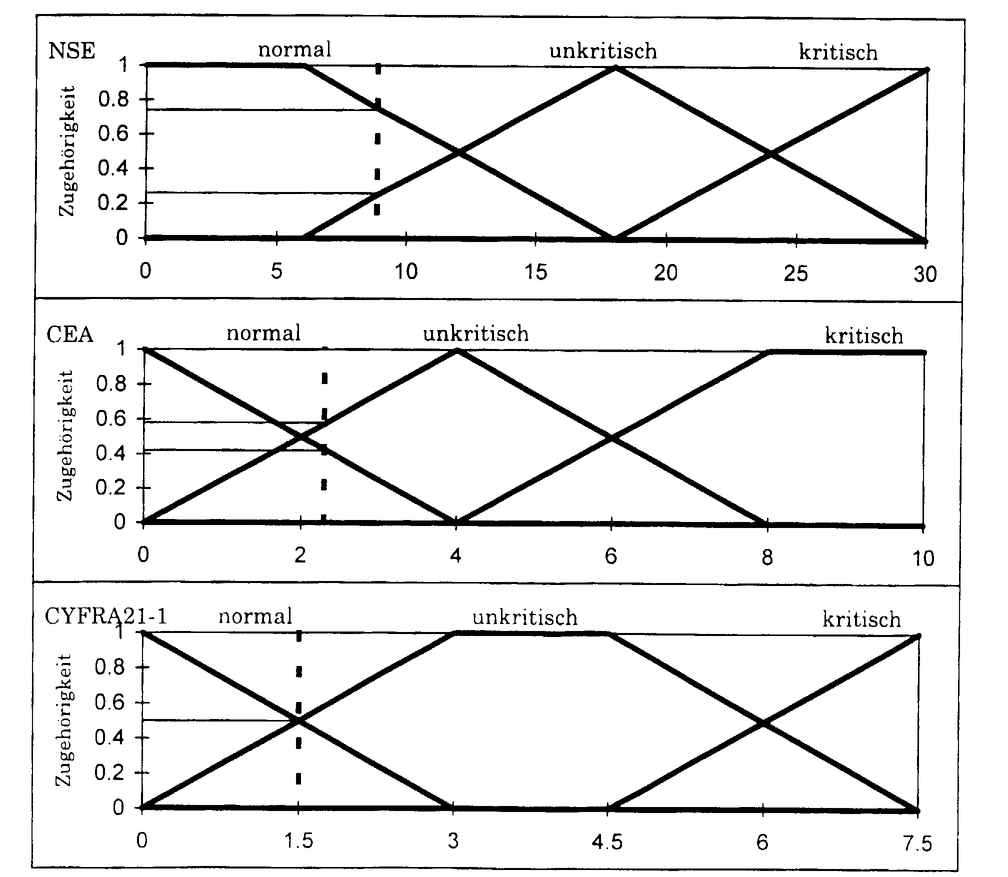 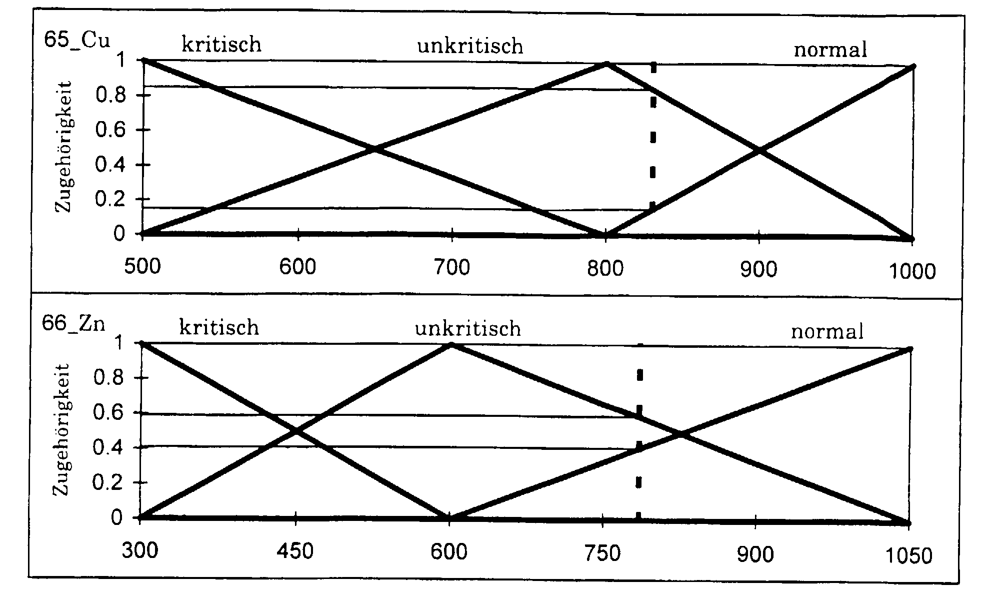
Im Ergebnis der Fuzzifizierung erhält man folgende (relativierte) Datenliste:

| Indikatorstoff | Meßwert in µg/l | Zugehörigkeit zu | | |
|---|---|---|---|---|
| | | "normal" | "unkritisch" | "kritisch" |
| NSE | 8.56 | 0.74 | 0.26 | 0.00 |
| CEA | 2.07 | 0.58 | 0.42 | 0.00 |
| CYFRA21-1 | 1.48 | 0.50 | 0.50 | 0.00 |
| 65_Cu | 841 | 0.15 | 0.85 | 0.00 |
| 66_Zn | 785 | 0.41 | 0.59 | 0.00 |

(3) Die so ermittelten fuzzifizierten Datenblöcke werden im letzten Verfahrensschritt mit den gespeicherten Datenblöcken verglichen, mittels Fuzzy-Entscheidungshilfesystem kombiniert, in ihren Wechselwirkungen ausgewertet und bezüglich jeder der Charakteristika "Malignität", "Histologie" und "Differentialhistologie" einem Zahlenwert zwischen 0 und 1 zugeordnet. Für dieses Zahlenbeispiel findet man:

| Charakteristik | Zugehörigkeit |
|---|---|
| Malignität | 0.04 |
| Histologie | 0.00 |
| Differentialhistologie | 0.00 |

### Ausführungsbeispiel 2:

(1) Die Meßwerte der Indikatorstoffe im Blutplasma einer Person werden gemäß erstem Verfahrensschritt in Datenblöcke zusammengefaßt. Im Ergebnis erhält man folgende Datenliste:

| Indikatorstoff | Meßwert |
|---|---|
| NSE (Neuronenspezifische Enolase) | 9.88 µg/l |
| CEA (Carzinoembryonales Antigen) | 2.44 µg/l |
| CYFRA21-1 (spezieller Tumormarker) | 7.42 µg/l |
| Kupfer-Konzentration (65_Cu) | 1605 µg/l |
| Zink-Konzentration (66_Zn) | 526 µg/l |

(2) Im zweiten Verfahrensschritt wird jeder Meßwert der Indikatorstoffe anhand gespeicherter, diagostisch gesicherter Datenblöcke fuzzifiziert, um so die Übereinstimmung mit den Referenzbeispielen in relativen Größen entsprechend der qualitativen Bewertungen "normal", "unkritisch" bzw. "kritisch" ausdrücken zu können: 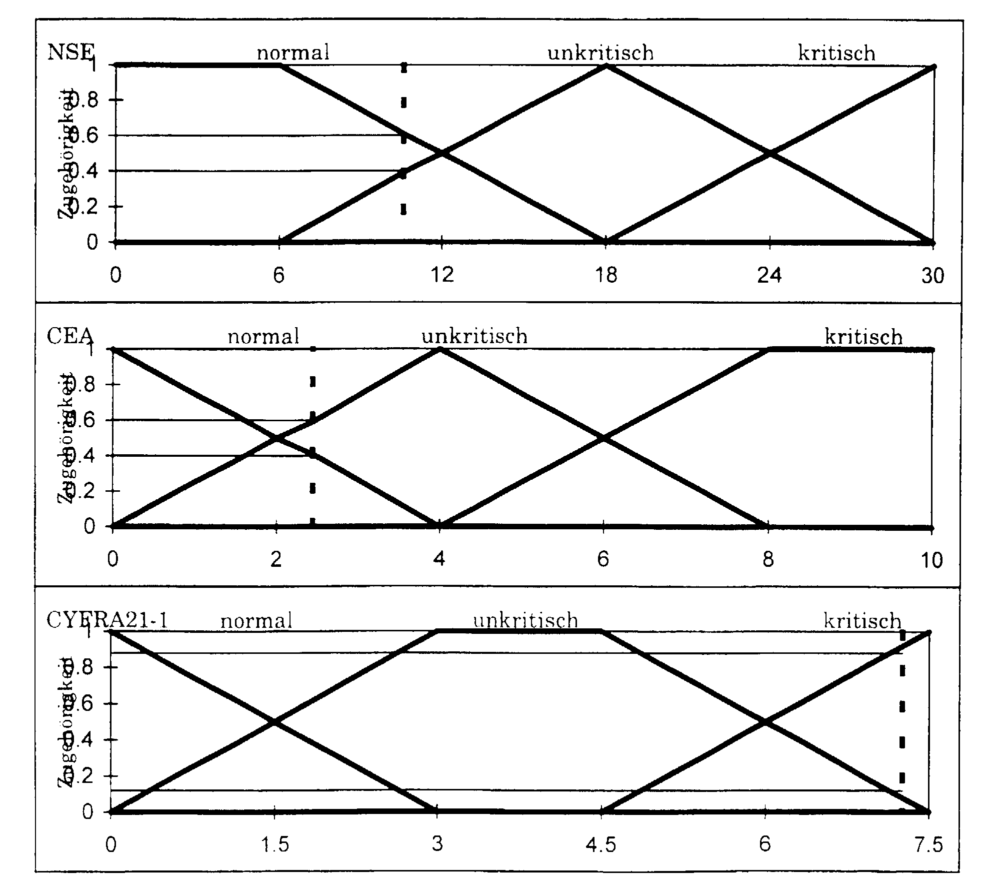 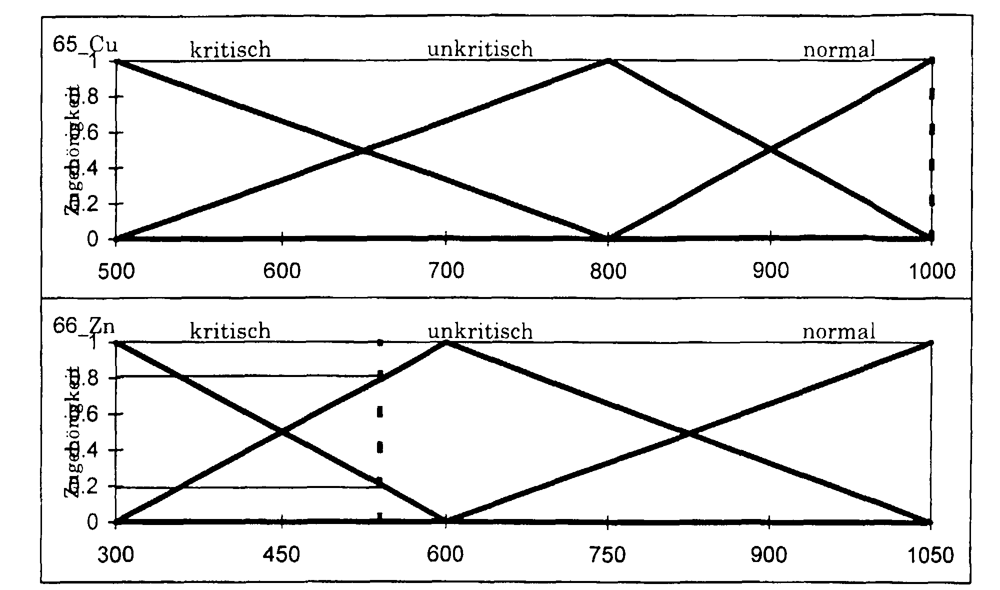
Im Ergebnis der Fuzzifizierung erhält man folgende (relativierte) Datenliste:

| Indikatorstoff | Meßwert in µg/l | Zugehörigkeit zu | | |
|---|---|---|---|---|
| | | "normal" | "unkritisch" | "kritisch" |
| NSE | 9.88 | 0.60 | 0.40 | 0.00 |
| CEA | 2.44 | 0.60 | 0.40 | 0.00 |
| CYFRA21-1 | 7.42 | 0.00 | 0.12 | 0.88 |
| 65_Cu | 1605 | 1.00 | 0.00 | 0.00 |
| 66_Zn | 526 | 0.00 | 0.81 | 0.19 |

(3) Die so ermittelten fuzzifizierten Datenblöcke werden im letzten Verfahrensschritt mit den gespeicherten Datenblöcken verglichen, mittels Fuzzy-Entscheidungshilfesystem kombiniert, in ihren Wechselwirkungen ausgewertet und bezüglich jeder der Charakteristika "Malignität", "Histologie" und "Differentialhistologie" einem Zahlenwert zwischen 0 und 1 zugeordnet. Für dieses Zahlenbeispiel findet man:

| Charakteristik | Zugehörigkeit |
|---|---|
| Malignität | 0.91 |
| Histologie | 0.72 |
| Differentialhistologie | 0.86 |

### Ausführungsbeispiel 3:

(1) Die Meßwerte der Indikatorstoffe im Blutplasma einer Person werden gemäß erstem Verfahrensschritt in Datenblöcke zusammengefaßt. Im Ergebnis erhält man folgende Datenliste:

| Indikatorstoff | Meßwert |
|---|---|
| NSE (Neuronenspezifische Enolase) | 9.07 µg/l |
| CEA (Carzinoembryonales Antigen) | >1100 µg/l |
| CYFRA21-1 (spezieller Tumormarker) | 49.90 µg/l |
| Kupfer-Konzentration (65_Cu) | 1329 µg/l |
| Zink-Konzentration (66_Zn) | 960 µg/l |

(2) Im zweiten Verfahrensschritt wird jeder Meßwert der Indikatorstoffe anhand gespeicherter, diagostisch gesicherter Datenblöcke fuzzifiziert, um so die Übereinstimmung mit den Referenzbeispielen in relativen Größen entsprechend der qualitativen Bewertungen "normal", "unkritisch" bzw. "kritisch" ausdrücken zu können: 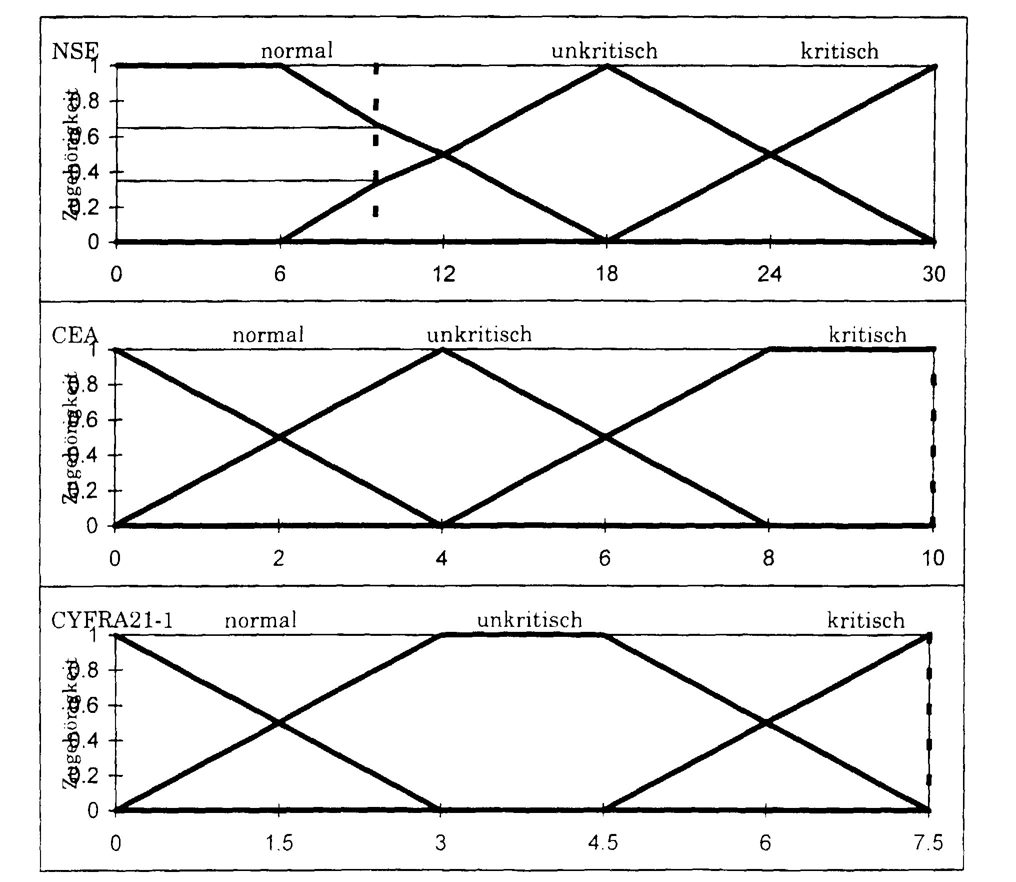 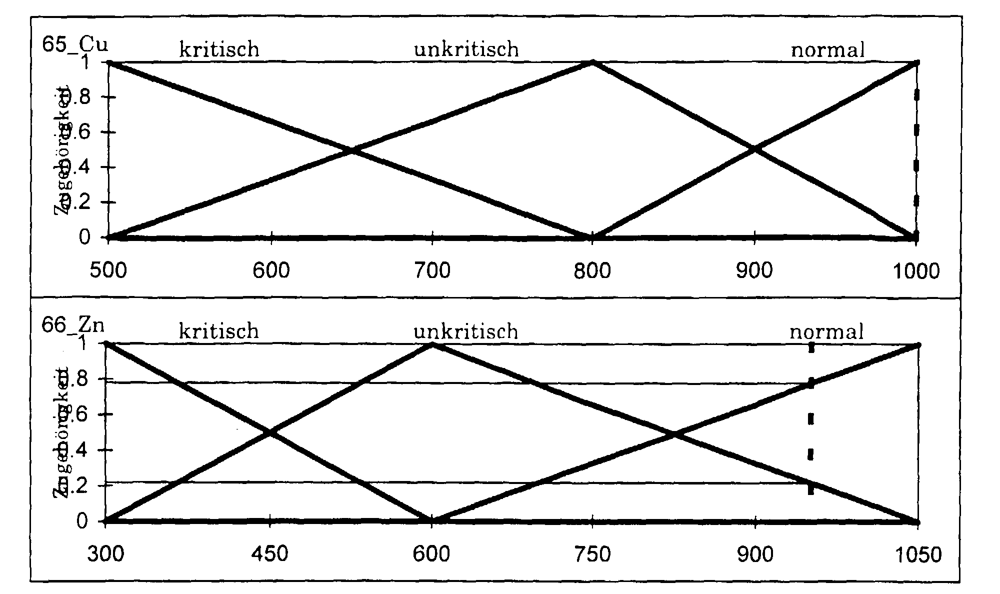
Im Ergebnis der Fuzzifizierung erhält man folgende (relativierte) Datenliste:

| Indikatorstoff | Meßwert in µg/l | Zugehörigkeit zu | | |
|---|---|---|---|---|
| | | "normal" | "unkritisch" | "kritisch" |
| NSE | 9.07 | 0.63 | 0.37 | 0.00 |
| CEA | >1100 | 0.00 | 0.00 | 1.00 |
| CYFRA21-1 | 49.90 | 0.00 | 0.00 | 1.00 |
| 65_Cu | 1329 | 1.00 | 0.00 | 0.00 |
| 66_Zn | 960 | 0.78 | 0.22 | 0.00 |

(3) Die so ermittelten fuzzifizierten Datenblöcke werden im letzten Verfahrensschritt mit den gespeicherten Datenblöcken verglichen, mittels Fuzzy-Entscheidungshilfesystem kombiniert, in ihren Wechselwirkungen ausgewertet und bezüglich jeder der Charakteristika "Malignität", "Histologie" und "Differentialhistologie" einem Zahlenwert zwischen 0 und 1 zugeordnet. Für dieses Zahlenbeispiel findet man:

| Charakteristik | Zugehörigkeit |
|---|---|
| Malignität | 0.94 |
| Histologie | 0.72 |
| Differentialhistologie | 0.18 |

### Ausführungsbeispiel 4:

(1) Die Meßwerte der Indikatorstoffe im Blutplasma einer Person werden gemäß erstem Verfahrensschritt in Datenblöcke zusammengefaßt. Im Ergebnis erhält man folgende Datenliste:

| Indikatorstoff | Meßwert |
|---|---|
| NSE (Neuronenspezifische Enolase) | 29.80 µg/l |
| CEA (Carzinoembryonales Antigen) | 1.37 µg/l |
| CYFRA21-1 (spezieller Tumormarker) | 3.47 µg/l |
| Kupfer-Konzentration (65_Cu) | 1590 µg/l |
| Zink-Konzentration (66_Zn) | 790 µg/l |

(2) Im zweiten Verfahrensschritt wird jeder Meßwert der Indikatorstoffe anhand gespeicherter, diagostisch gesicherter Datenblöcke fuzzifiziert, um so die Übereinstimmung mit den Referenzbeispielen in relativen Größen entsprechend der qualitativen Bewertungen "normal", "unkritisch" bzw. "kritisch" ausdrücken zu können: 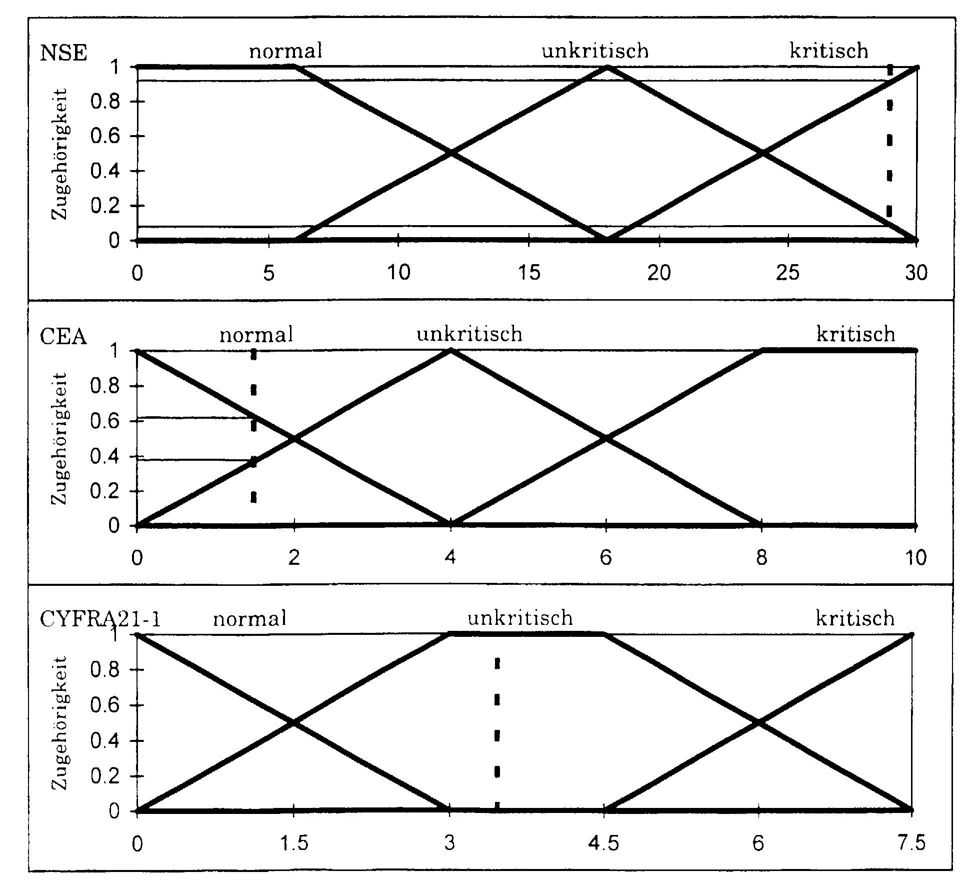 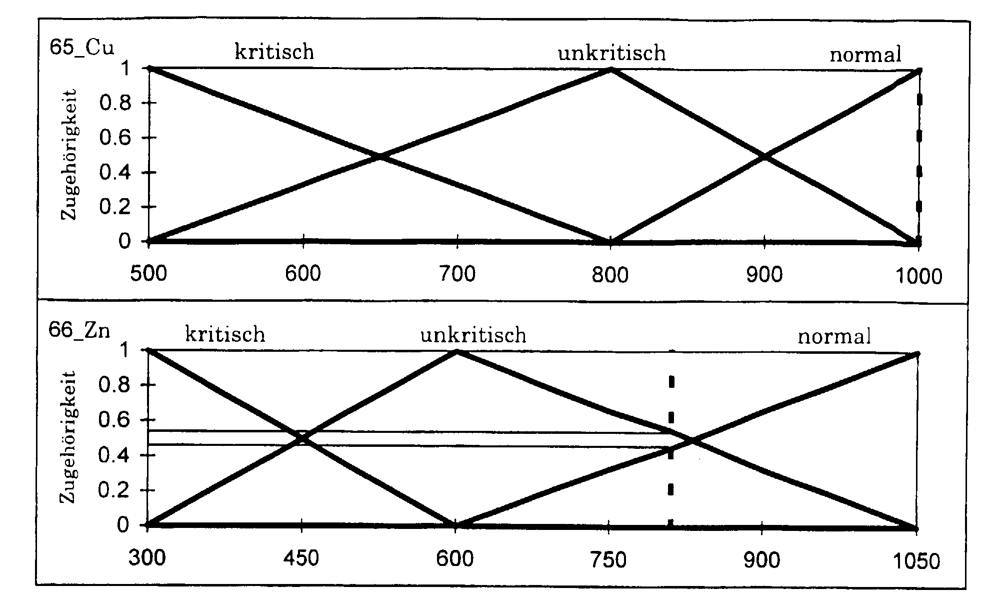
Im Ergebnis der Fuzzifizierung erhält man folgende (relativierte) Datenliste:

| Indikatorstoff | Meßwert | Zugehörigkeit zu | | |
|---|---|---|---|---|
| | in µg/l | "normal" | "unkritisch" | "kritisch" |
| NSE | 29.80 | 0.00 | 0.08 | 0.92 |
| CEA | 1.37 | 0.62 | 0.38 | 0.00 |
| CYFRA21-1 | 3.47 | 0.00 | 1.00 | 0.00 |
| ... | ... | | ... | ... |
| 65_Cu | 1590 | 1.00 | 0.00 | 0.00 |
| 66_Zn | 790 | 0.46 | 0.54 | 0.00 |

(3) Die so ermittelten fuzzifizierten Datenblöcke werden im letzten Verfahrensschritt mit den gespeicherten Datenblöcken verglichen, mittels Fuzzy-Entscheidungshilfesystem kombiniert, in ihren Wechselwirkungen ausgewertet und bezüglich jeder der Charakteristika "Malignität", "Histologie" und "Differentialhistologie" einem Zahlenwert zwischen 0 und 1 zugeordnet. Für dieses Zahlenbeispiel findet man:

| Charakteristik | Zugehörigkeit |
|---|---|
| Malignität | 0.90 |
| Histologie | 0.49 |
| Differentialhistologie | 0.00 |

## Patentansprüche

1. Verfahren zur Ermittlung signifikanter Abweichungen des Zellenwachstums anhand gefundener Indikatorstoffe aus Körperflüssigkeiten, dadurch gekennzeichnet, daß für die nachfolgenden Verfahrensschritte Indikatorstoffe verwendet werden, die je nach ihrer Art von Tumorzellen produziert, in anderen Körperzellen durch den Tumor induziert und als tumorunspezifische Stoffe in ihrer Konzentration durch den Tumor verändert werden, sowie Meßwerte der Indikatorstoffe verwendet werden, die aus einer direkten Stoffanalyse ermittelt worden sind, und daß
(1) die Meßwerte der Indikatorstoffe in Datenblöcke zusammengefaßt werden,
(2) jeder Meßwert der Indikatorstoffe anhand gespeicherter und diagnostisch gesicherter Datenblöcke fuzzifiziert wird, um so eine Übereinstimmung in relativen Größen entsprechend der qualitativen Bewertung "normal", "unkritisch", "kritisch" ausdrücken zu können, und
(3) diese fuzzifizierten Datenblöcke mit den gespeicherten Datenblöcken verglichen, mittels Fuzzy-Entscheidungshilfesystem kombiniert und bezüglich der Charakteristika
. Malignität
. Histologie
. Differentialhistologie
einem Zahlenwert zwischen 0 und 1 zugeordnet werden.

2. Verfahren nach Anspruch 1, dadurch gekennzeichnet, daß die Indikatorstoffe Makromoleküle und Spurenelemente sind.

3. Verfahren nach Anspruch 1, dadurch gekennzeichnet, daß die Indikatorstoffe Spurenelemente sind.

4. Verfahren nach Anspruch 1, dadurch gekennzeichnet, daß die Indikatorstoffe Makromoleküle sind.

## Claims

1. Methods for determining significant deviations of the cell growth with the help of found body fluid indicator substances, characterised by the fact that indicator substances are used for the subsequent process steps, which, depending on their type, are produced by tumour cells, induced in other body cells by the tumour and changed as tumourunspecific substances in their concentration by the tumour, as well as that measuring values for the indicator substances are used, which have been determined from a direct substance analysis, and that
(1) the measuring values for the indicator substances are combined in uata blocks,
(2) each measuring value for the indicator substances is subjected to a fuzzy procedure based on stored and diagnostically verified data blocks so as to be able to express an agreement of relative variables in accordance with the qualitative assessment as being "normal", uncritical", critical", and
(3) these fuzzied data blocks are compared with the stored data blocks, combined by means of the fuzzy decision support system, and assigned with respect to the characteristics
. malignancy
. histology histology
. differential histology
to a numerical value between 0 and 1.

2. The methods of claim 1, characterised by the fact that the indicator substances are macromolecules and trace elements.

3. The methods of claim 1, characterised by the fact that the indicator substances are trace elements.

4. The methods of claim 1, characterised by the fact that the indicator substances are macromolecules.

## Revendications

1. Procédé de détermination des aberrations significatives dans la croissance de cellules à l'aide des agents indicateurs découverts dans le volume humoral caractérisé en ce que, dans la suite du procédé, sont utilisés de tels agents indicateurs qui, selon leur nature, seront produits par les cellules tumorales, induits dans d'autres cellules du corps par la tumeur et modifiés par la tumeur dans leur concentration en agents de tumeur atypiques et sont utilisées de telles valeurs mesurées des agents indicateurs qui seront déterminées à partir d'une analyse immédiate des agents et en ce que
(1) les valeurs mesurées des agents indicateurs sont réunies en blocs de données,
(2) chaque valeur mesurée des agents indicateurs est traitée selon la logique Fuzzy à l'aide des blocs de données enregistrés et établis par diagnostique pour ainsi pouvoir traduire une concordance avec des grandeurs relatives et conformément à l'évaluation qualitative suivante : "normale", "non critique" et "critique".
(3) Ces blocs de données traités selon la logique Fuzzy sont comparés avec les blocs de données enregistrés, ensuite combinés à l'aide du système de décision Fuzzy et attribués à une valeur numérique qui se situe entre 0 et 1 pour les caractéristiques suivantes :
. malignité
. histologie
. histologie différentielle.

2. Procédé selon la revendication 1, caractérisé en ce que les agents indicateurs sont des macromolécules et oligoéléments.

3. Précédé selon la revendication 1, caractérisé en ce que les agents indicateurs sont des oligoéléments.

4. Procédé selon la revendication 1, caractérisé en ce que les agents indicateurs sont des macromolécules.
